# EUROPEAN PATENT APPLICATION

(11) **EP 4 067 279 A1**
(43) Date of publication of application: **05.10.2022**
(21) Application number: 21210087.9
(22) Date of filing: 24.11.2021
(51) Int. Cl.: B66B 1/46, B66B 23/24

(54) **SELF-SANITIZING PASSENGER CONTACT COMPONENT FOR ELEVATOR, ESCALATOR OR MOVING WALKWAY**

(30) Priority: 01.04.2021 US 202117219935
(71) Applicant: Otis Elevator Company, Farmington, Connecticut 06032 (US)
(72) Inventor: Guilani, Brad, Farmington (US)
(74) Representative: Dehns

(57) **Abstract**

Disclosed is an uncoated passenger contact component including a self-sanitizing polymer composition.

## Description

### BACKGROUND

Exemplary embodiments pertain to the art of elevator, escalator, and/or moving walkway surfaces.

A variety of processes and solutions have been proposed and are being used to help prevent the spread of communicable diseases due to passengers' interaction with fixtures and buttons of an elevator, escalator, and/or moving walkway. These include use of disinfecting chemicals and ultra-violet (UV) light that typically treat contact surfaces. While effective, improvements are sought.

### BRIEF DESCRIPTION

Disclosed is an uncoated passenger contact component including a self-sanitizing polymer composition wherein the contact surface (or the passenger contact component) is part of an elevator, escalator, or moving walkway (e.g. an elevator, escalator, or moving walkway as disclosed herein).

Also disclosed is an elevator, escalator or moving walkway comprising an uncoated passenger contact component comprising a self-sanitizing polymer composition.

In addition to one or more of the features described above, or as an alternative to any of the foregoing embodiments, the self-sanitizing polymer composition may include a polymer with a plurality of covalently bound biocidal functional groups.

In addition to one or more of the features described above, or as an alternative to any of the foregoing embodiments, the plurality of covalently bound biocidal functional groups may include photosensitizing functional groups. The photosensitizing functional groups may include a porphyrin or phthalocyanine derivative.

In addition to one or more of the features described above, or as an alternative to any of the foregoing embodiments, the plurality of covalently bound biocidal functional groups may include anionic functional groups. The anionic functional groups may include sulfinate anions, sulfonate anions, phosphonate anions, or a combination thereof.

In addition to one or more of the features described above, or as an alternative to any of the foregoing embodiments, the uncoated passenger contact component may be a control button, a control panel, a support rail, a destination entry device, or a combination of the foregoing.

In addition to one or more of the features described above, or as an alternative to any of the foregoing embodiments, the self-sanitizing polymer composition may include a self-sanitizing polymer having a glass transition temperature (Tg) greater than 45°C and less than or equal to 250°C.

In addition to one or more of the features described above, or as an alternative to any of the foregoing embodiments, the (self-sanitizing) polymer may be a block copolymer or the self-sanitizing polymer composition may comprise a block copolymer. The (self-sanitizing) polymer may be a thermoplastic elastomer or the self-sanitizing polymer composition may comprise a thermoplastic elastomer. The (self-sanitizing) polymer may be a styrenic polymer or the self-sanitizing polymer composition may comprise a styrenic polymer.

Also disclosed is an elevator including an uncoated passenger contact component (e.g. an uncoated passenger contact component as disclosed herein) including a self-sanitizing polymer composition (e.g. a self-sanitizing polymer composition as herein described).

In addition to one or more of the features described above, or as an alternative to any of the foregoing embodiments, the self-sanitizing polymer composition may include a polymer with a plurality of covalently bound biocidal functional groups.

In addition to one or more of the features described above, or as an alternative to any of the foregoing embodiments, the plurality of covalently bound biocidal functional groups may include photosensitizing functional groups. The photosensitizing functional groups may include a porphyrin or phthalocyanine derivative.

In addition to one or more of the features described above, or as an alternative to any of the foregoing embodiments, the plurality of covalently bound biocidal functional groups may include anionic functional groups. The anionic functional groups may include sulfinate anions, sulfonate anions, phosphonate anions, or a combination thereof.

Also disclosed is an escalator or moving walkway comprising an uncoated passenger contact component (e.g. an uncoated passenger contact component as disclosed herein) comprising a self-sanitizing polymer composition (e.g. a self-sanitizing polymer composition as herein described).

In some embodiments, the self-sanitizing polymer composition comprises a polymer with a plurality of covalently bound biocidal functional groups.

In some embodiments, the plurality of covalently bound biocidal functional groups comprise photosensitizing functional groups, anionic functional groups, or a combination thereof.

### BRIEF DESCRIPTION OF THE DRAWINGS

The following descriptions should not be considered limiting in any way. With reference to the accompanying drawings, like elements are numbered alike:
FIG. 1 shows an elevator control panel;
FIG. 2 shows an elevator request panel;
FIG. 3 shows an escalator having a passenger contact component; and
FIG. 4 shows a moving walkway or escalator passenger contact component.

### DETAILED DESCRIPTION

A detailed description of one or more embodiments of the disclosed apparatus and method are presented herein by way of exemplification and not limitation with reference to the Figures.

Current solutions to sanitizing passenger contact components of an elevator, escalator or moving walkway include contacting the surfaces with disinfecting chemicals, bathing the contact surfaces with ultraviolet light, coating the surfaces with a biocidal coating, or including a biocidal additive in a composition. These solutions all suffer from drawbacks. Contacting the surfaces with disinfecting chemicals is labor intensive and subject to human error. Bathing the contact surfaces with ultraviolet light requires the installation of equipment and can be labor intensive. Additionally, repeated applications of ultraviolet light can have a negative effect on the materials used to form the contact surface. Biocidal coatings are subject to wear through and will eventually disappear from the most often touched surfaces. Biocidal additives are similarly worn off of the contact surface. A more long-lived solution is desired.

This need is addressed by an uncoated passenger contact component formed from a self-sanitizing polymer composition. An uncoated passenger contact component is defined as having no coating applied to the surface formed from the self-sanitizing polymer composition. The self-sanitizing polymer composition includes a self-sanitizing polymer that has a plurality of covalently bound biocidal functional groups. The covalently bound biocidal functional groups may include photosensitizing groups, anionic functional groups, or a combination thereof. The self-sanitizing polymer may have a glass transition temperature (Tg) greater than 45°C and less than or equal to 250°C. Alternatively, when the self-sanitizing polymer comprises a thermoplastic elastomer the glass transition temperature (Tg) may be below 0°C.

. The self-sanitizing polymer may be a thermoplastic or a thermoset. The self-sanitizing polymer may be a block copolymer. The self-sanitizing polymer may be a thermoplastic elastomer. The self-sanitizing polymer may be a styrenic polymer such as polystyrene, copolymers including polystyrene subunits and mixtures such as polycarbonate/acrylonitrile-butadiene-styrene polymer (PC/ABS).

In some embodiments, the plurality of covalently bound biocidal functional group includes photosensitizing functional groups. The photosensitizing groups, when exposed to visible light, generates singlet oxygen which leads to the destruction of pathogens present on the surface. By covalently bonding the photosensitizing group to the polymer, there is no need for a component of the composition to migrate to the surface and no potential for the photosensitizing group to be worn off. Exemplary photosensitizing groups include porphyrin, phthalocyanine, and phthalocyanine derivatives. The photosensitizing groups may be present in an amount greater than or equal to 0.5 weight percent (wt%), or greater than or equal to 1 wt%, based on the total weight of the self-sanitizing polymer. Polymers having a phthalocyanine or phthalocyanine derivative covalently bound to the polymer are disclosed in U.S. Patent No. 8,741,264, which is incorporated by reference herein in its entirety.

In some embodiments, the plurality of covalently bound biocidal functional group includes anionic functional groups. Upon exposure to ambient moisture, the anionic functional groups create a sudden and drastic change in pH on the uncoated passenger contact component which promotes destruction of a pathogen. Exemplary anionic functional groups include a sulfinate anion, a sulfonate anion, and a phosphonate anion. The self-sanitizing polymer may include different types of anionic functional groups. The anionic functional group may be present in an amount of at least 20 mol%. Exemplary self-sanitizing polymers that have a covalently bound anionic functional group are described in "Inherently self-sterilizing charged multiblock polymers that kill drug-resistant microbes in minutes" by Peddinti et al. and published in Issue 10. 2019 of Materials Horizons.

In some embodiments, the self-sanitizing polymer is a multiblock copolymer having anionic functional groups located on a midblock in sufficient quantity to result in anti-microbial activity. One example is a TESET polymer having at least 20 mol%, or at least 40 mol%, or at least 50 mol% of a sulfonate anionic functional group. TESET is an acronym for a pentablock copolymer also known as poly[t-butylstyrene-b-(ethylene-alt-propylene)-b-(styrenesulfonate)-b-(ethylene-alt-propylene)-b-t-butylstyrene]. Other multiblock copolymers include the TST triblock copolymer (poly(p-tert-butylstyrene-b-styrene-b-p-tert-butylstyrene)) in which the midblock is sulfonated in an amount of at least 20mol%, or at least 40 mol%.

In addition to the self-sanitizing polymer, the self-sanitizing polymer composition may further include pigments, dyes, surfactants, molding additives, viscosity modifiers, or other additives known in the art for injection molded or extruded polymer compositions.

The uncoated passenger contact component may be formed from a self-sanitizing polymer composition by injection molding, extrusion, compression molding, vacuum molding or other molding technique known in the art as suitable for polymer compositions. Because the biocidal activity of the polymer is due to a covalently bound terminal group there is little concern about destroying or deactivating the biocidal activity when forming the contact surface.

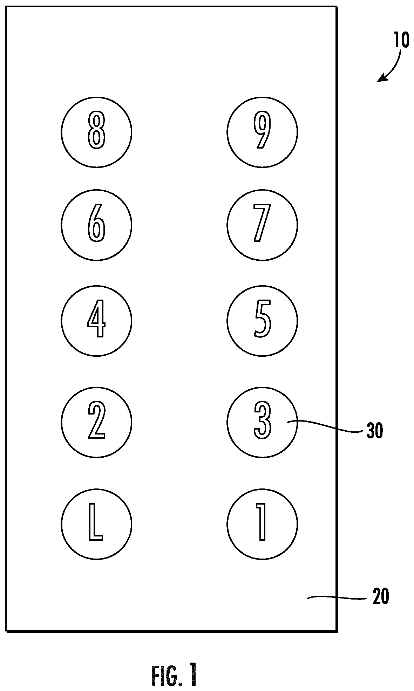

Turning now to the Figures, FIG. 1 shows an elevator control panel 10 having a panel plate 20 and control buttons 30. The panel plate, control buttons or both can be considered a passenger contact component and formed from the self-sanitizing polymer.

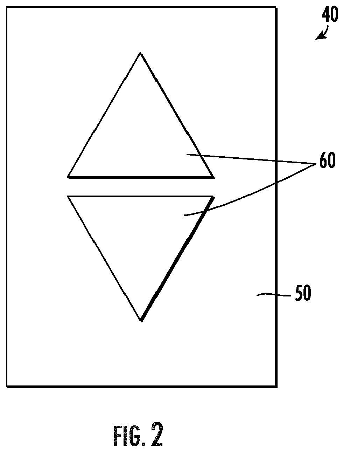

FIG. 2 shows an elevator request panel 40 having a panel plate 50 and elevator request buttons 60. The panel plate and elevator request buttons can be considered passenger contact components.

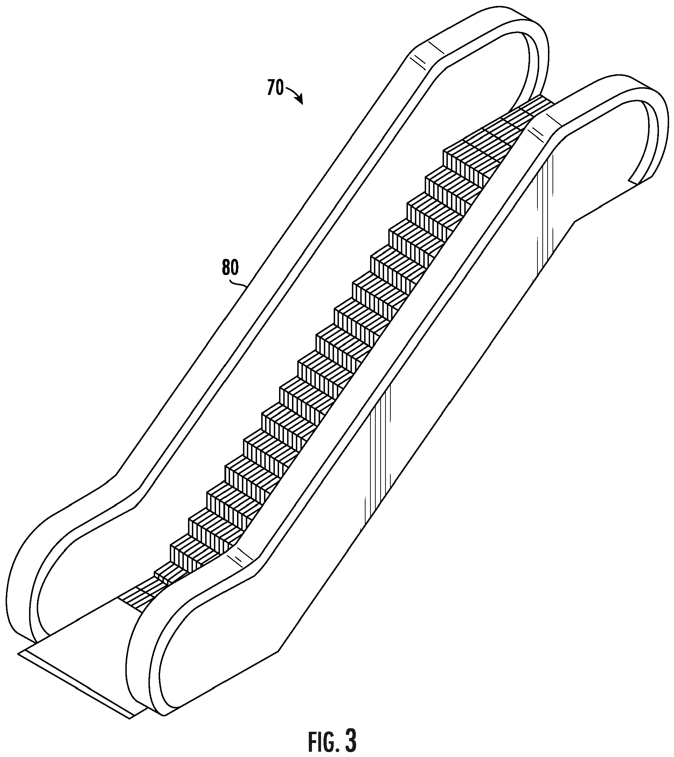

FIG. 3 shows an escalator 70 having a handrail 80 which is a passenger contact component.

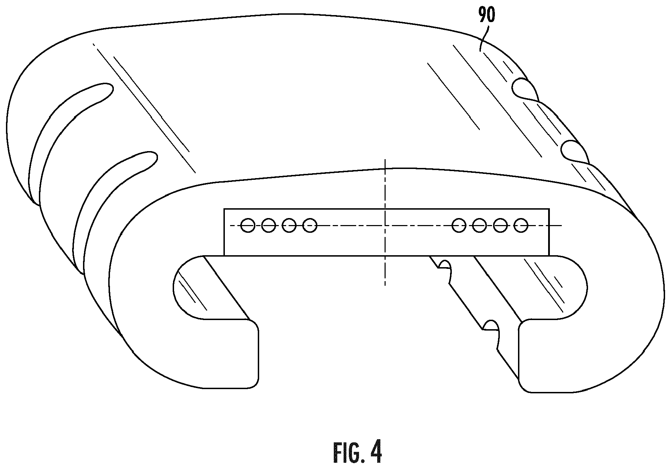

FIG. 4 shows a moving walkway or escalator passenger contact component 90.

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof.

While the present disclosure has been described with reference to an exemplary embodiment or embodiments, it will be understood by those skilled in the art that various changes may be made and equivalents may be substituted for elements thereof without departing from the scope of the present disclosure. In addition, many modifications may be made to adapt a particular situation or material to the teachings of the present disclosure without departing from the essential scope thereof. Therefore, it is intended that the present disclosure not be limited to the particular embodiment disclosed as the best mode contemplated for carrying out this present disclosure, but that the present disclosure will include all embodiments falling within the scope of the claims.

Certain embodiments of the present disclosure are as follows:
1. An uncoated passenger contact component comprising a self-sanitizing polymer composition, wherein the passenger contact component is part of an elevator, escalator or moving walkway.
2. The uncoated passenger contact component of embodiment 1, wherein the self-sanitizing polymer composition comprises a polymer with a plurality of covalently bound biocidal functional groups.
3. The uncoated passenger contact component of embodiment 2, wherein the plurality of covalently bound biocidal functional groups comprise photosensitizing functional groups.
4. The uncoated passenger contact component of embodiment 3, wherein the photosensitizing functional groups comprise a porphyrin or phthalocyanine derivative.
5. The uncoated passenger contact component of embodiment 2, wherein the plurality of covalently bound functional groups comprise anionic functional groups.
6. The uncoated passenger contact component of embodiment 5, wherein the anionic functional groups comprise sulfinate anions, sulfonate anions, phosphonate anions, or a combination thereof.
7. The uncoated passenger contact component of embodiment 2, wherein the polymer is a block copolymer.
8. The uncoated passenger contact component of embodiment 2, wherein the polymer is a thermoplastic elastomer.
9. The uncoated passenger contact component of embodiment 2, wherein the polymer is a styrenic polymer.
10. The uncoated passenger contact component of embodiment 1, wherein the uncoated passenger contact component is a control button, a control panel, a support rail, a destination entry device, or a combination of the foregoing.
11. The uncoated passenger contact component of embodiment 1, wherein the self-sanitizing polymer composition includes a self-sanitizing polymer having a glass transition temperature (Tg) greater than 45°C and less than or equal to 250°C.
12. An elevator comprising an uncoated passenger contact component comprising a self-sanitizing polymer composition.
13. The elevator of embodiment 12, wherein the self-sanitizing polymer composition comprises a polymer with a plurality of covalently bound biocidal functional groups.
14. The elevator of embodiment 13, wherein the plurality of covalently bound biocidal functional groups comprise photosensitizing functional groups.
15. The elevator of embodiment 14, wherein the photosensitizing functional groups comprise a porphyrin or phthalocyanine derivative.
16. The elevator of embodiment 13, wherein the plurality of covalently bound biocidal functional groups comprise anionic functional groups.
17. The uncoated elevator of embodiment 16, wherein the anionic functional groups comprise sulfinate anions, sulfonate anions, phosphonate anions, or a combination thereof.
18. An escalator or moving walkway comprising an uncoated passenger contact component comprising a self-sanitizing polymer composition.
19. The escalator or moving walkway of embodiment 18, wherein the self-sanitizing polymer composition comprises a polymer with a plurality of covalently bound biocidal functional groups.
20. The escalator or moving walkway of embodiment 18, wherein the plurality of covalently bound biocidal functional groups comprise photosensitizing functional groups, anionic functional groups, or a combination thereof.

## Claims

1. An uncoated passenger contact component comprising a self-sanitizing polymer composition, wherein the passenger contact component is part of an elevator, escalator or moving walkway.

2. The uncoated passenger contact component of claim 1, wherein the self-sanitizing polymer composition comprises a polymer with a plurality of covalently bound biocidal functional groups.

3. The uncoated passenger contact component of claim 2, wherein the plurality of covalently bound biocidal functional groups comprise photosensitizing functional groups.

4. The uncoated passenger contact component of claim 3, wherein the photosensitizing functional groups comprise a porphyrin or phthalocyanine derivative.

5. The uncoated passenger contact component of any one of claims 2-4, wherein the plurality of covalently bound biocidal functional groups comprise anionic functional groups.

6. The uncoated passenger contact component of claim 5, wherein the anionic functional groups comprise sulfinate anions, sulfonate anions, phosphonate anions, or a combination thereof.

7. The uncoated passenger contact component of any one of claims 2-6, wherein the polymer is a block copolymer.

8. The uncoated passenger contact component of any one of claims 2-7, wherein the polymer is a thermoplastic elastomer.

9. The uncoated passenger contact component of any one of claims 2-8, wherein the polymer is a styrenic polymer.

10. The uncoated passenger contact component of any one of the preceding claims, wherein the uncoated passenger contact component is a control button, a control panel, a support rail, a destination entry device, or a combination of the foregoing.

11. The uncoated passenger contact component of any one of the preceding claims, wherein the self-sanitizing polymer composition includes a self-sanitizing polymer having a glass transition temperature (Tg) greater than 45°C and less than or equal to 250°C.

12. An elevator, escalator or moving walkway comprising an uncoated passenger contact component comprising a self-sanitizing polymer composition.

13. The elevator, escalator or moving walkway of claim 12, wherein the self-sanitizing polymer composition comprises a polymer with a plurality of covalently bound biocidal functional groups.

14. The elevator, escalator or moving walkway of claim 13, wherein the plurality of covalently bound biocidal functional groups comprise photosensitizing functional groups, optionally wherein the photosensitizing functional groups comprise a porphyrin or phthalocyanine derivative.

15. The elevator, escalator or moving walkway of claim 13 or claim 14, wherein the plurality of covalently bound biocidal functional groups comprise anionic functional groups, optionally wherein the anionic functional groups comprise sulfinate anions, sulfonate anions, phosphonate anions, or a combination thereof.
